(19) Europäisches Patentamt
European Patent Office
Office européen des brevets

(11) Veröffentlichungsnummer: **0 031 297**
**A2**

(12) # EUROPÄISCHE PATENTANMELDUNG

(21) Anmeldenummer: **80810372.5**

(22) Anmeldetag: **01.12.80**

(51) Int. Cl.³: **C 07 D 207/416**
A 01 N 43/36, C 07 D 405/12

(30) Priorität: **07.12.79 CH 10871/79**

(43) Veröffentlichungstag der Anmeldung:
**01.07.81 Patentblatt 81/26**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Nyfeler, Robert, Dr.**
**Bärenfelserstrasse 8**
**CH-4057 Basel(CH)**

(54) **Pyrrolidin-2,5-dion-Derivate, Verfahren zu ihrer Herstellung und Verwendung als mikrobiozide Mittel.**

(57) Es werden neue Verbindungen der hierin definierten Formel I

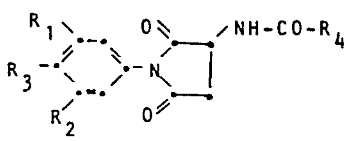

(I),

beschrieben, die wertvolle mikrobizide Eigenschaften aufweisen. Sie lassen sich zur Bekämpfung von pflanzenschädlichen Mikroorganismen, insbesondere gegen phytopathogene Pilze verwenden. So besitzen die Verbindungen der Formel I eine für praktische Bedürfnisse sehr günstige kurative, präventive und systemische Wirkung zum Schutze von Kulturpflanzen, ohne diese durch unerwünschte Nebenwirkungen zu beeinflussen. Sie lassen sich in der Praxis allein oder in Form von Schädlingsbekämpfungsmittel anwenden.

EP 0 031 297 A2

Croydon Printing Company Ltd.

CIBA-GEIGY AG
Basel (Schweiz)

BEZEICHNUNG GEÄNDERT
siehe Titelseite

5-12624/=

Mikrobizide Mittel

Die vorliegende Erfindung betrifft Verbindungen der Formel I

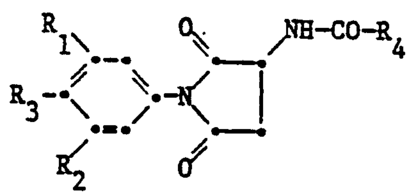

(I),

worin $R_1$ für Chlor, Brom oder Nitro, $R_2$ für Chlor oder Brom, $R_3$ für Wasserstoff oder Fluor stehen, $R_4$ ein gegebenenfalls durch Halogen oder $C_1$-$C_3$-Alkoxy substituiertes $C_1$-$C_9$-Alkyl, ein gegebenenfalls durch Halogen oder Aryl substituiertes $C_2$-$C_7$-Alkenyl, $C_2$-$C_4$-Alkinyl, $C_3$-$C_6$-Cycloalkyl, ein gegebenenfalls durch Halogen, Nitro oder $C_1$-$C_4$-Alkyl substituiertes Phenyl, einen 5-gliedrigen Heterocyclus mit einem Heteroatom oder -X-$R_5$ bedeutet, wobei X für Sauerstoff oder Schwefel steht und $R_5$ ein gegebenenfalls durch Halogen oder $C_1$-$C_3$-Alkoxy substituiertes $C_1$-$C_6$-Alkyl oder substutiertes $C_3$-$C_6$-Alkenyl, ein gegebenenfalls durch Halogen, Nitro, $C_1$-$C_3$-Alkyl oder $C_1$-$C_3$-Alkoxy substituiertes Phenyl oder ein gegebenenfalls durch Halogen oder Nitro substituiertes Benzyl bedeutet.

Unter Alkyl oder Alkylanteil eines anderen Substituenten sind je nach Zahl der angegebenen Kohlenstoffatome beispielsweise folgende Gruppen zu verstehen: Methyl, Aethyl, Propyl, Butyl, Pentyl, Hexyl oder Heptyl sowie ihre Isomeren, wie z.B. Isopropyl, Isobutyl, tert.-Butyl, Isopentyl usw. Alkenyl steht z.B. für Propenyl-(1), Allyl, Butenyl-(1), Butenyl-(2), Butenyl-(3) usw. Alkinyl steht beispielsweise für Aethinyl, Propinyl-(1), Propargyl, Butinyl-(1), usw. Unter Halogen soll hier und im folgenden Fluor, Chlor, Brom oder Jod verstanden werden, vorzugsweise Chlor oder Brom. Beispiele für

fünfgliedrige Heterocyclen mit einem Heteroatom sind: Tetrahydrofuran, Furan, Thiophen, Tetrahydrothiophen, Pyrrol, Pyrrolidin, Pyrrolin usw.

Die Verbindungen der Formel I lassen sich dabei in drei Hauptgruppen einteilen:

a) Verbindungen in denen der Rest $R_4$ direkt an die Carbamidgruppe gebunden ist - die Carbamide,

b) Verbindungen in denen der Rest $R_5$ über ein Sauerstoffatom an die Carbamidgruppe gebunden ist - die Carbamate,

c) Verbindungen in denen der Rest $R_5$ über ein Schwefelatom an die Carbamidgruppe gebunden ist - die Thiocarbamate.

Verbindungen der Formel I zeigen ein sehr wertvolles Mikrobizid-Spektrum. Sie lassen sich z.B. gegen phytopathogene Mikroorganismen, insbesondere gegen Pilze einsetzen.

Mikrobizide der Formel I mit folgenden Substituenten-typen sind bevorzugt:

Bei $R_1$, $R_2$ : Chlor

Bei $R_3$ : Wasserstoff

Bei $R_4$ : gegebenenfalls durch Halogen substituiertes $C_1$-$C_4$-Alkyl, gegebenenfalls durch Halogen oder Aryl substituiertes $C_2$-$C_7$-Alkenyl, gegebenenfalls durch Halogen oder $C_1$-$C_2$-Alkyl substituiertes Phenyl

Bei X : Sauerstoff, Schwefel

Bei $R_5$ : gegebenenfalls durch Halogen oder $C_1$-$C_3$-Alkoxy substituiertes $C_1$-$C_6$-Alkyl, $C_3$-$C_6$-Alkenyl, gegebenenfalls durch Halogen oder $C_1$-$C_3$-Alkyl substituiertes Phenyl.

Eine bevorzugte Gruppe von Mikrobiziden besteht aus Verbindungen der Formel I, worin $R_1$ und $R_2$ für Chlor stehen und $R_4$ ein gege-

benenfalls durch Halogen oder Aryl substituiertes $C_2$-$C_7$-Alkenyl bedeutet.

Eine weitere bevorzugte Gruppe von Mikrobiziden besteht aus Verbindungen der Formel I, worin $R_1$ und $R_2$ für Chlor stehen, $R_3$ Wasserstoff darstellt und $R_4$ für $-OR_5$ steht, wobei $R_5$ ein gegebenenfalls durch Halogen oder $C_1$-$C_3$-Alkoxy substituiertes $C_1$-$C_6$-Alkyl bedeutet.

Eine dritte bevorzugte Gruppe von Mikrobiziden besteht aus Verbindungen der Formel I, worin $R_1$ und $R_2$ für Chlor stehen, $R_3$ Wasserstoff bedeutet und $R_4$ für $-SR_5$ steht, wobei $R_5$ ein gegebenenfalls durch Halogen oder $C_1$-$C_3$-Alkoxy substituiertes $C_1$-$C_6$-Alkyl bedeutet.

Ferner werden folgende Einzelverbindungen besonders bevorzugt:

3-(N-Methoxycarbonylamino)-1-(3,5-dichlorphenyl)-pyrrolidin-2,5-dion,
3-(N-Äthoxycarbonylamino)-1-(3,5-dichlorphenyl)-pyrrolidin-2,5-dion,
3-(N-Trifluoracetylamino)-1-(3,5-dichlorphenyl)-pyrrolidin-2,5-dion,
3-[N-(2-Methyl-1-propenyl)-carbonylamino]-1-(3,5-dichlorphenyl)-pyrrolidin-2,5-dion,
3-[N-(1,3-Pentadienyl)-carbonylamino]-1-(3,5-dichlorphenyl)-pyrrolidin-2,5-dion,
3-(N-Cyclopropylcarbonylamino)-1-(3,5-dichlorphenyl)-pyrrolidin-2,5-dion,
3-(N-Isobutyloxycarbonylamino)-1-(3,5-dichlorphenyl)-pyrrolidin-2,5-dion,
3-[N-(2-Propenyloxy)-carbonylamino]-1-(3,5-dichlorphenyl)-pyrrolidin-2,5-dion,
3-[N-(2-Bromäthoxy)-carbonylamino]-1-(3,5-dichlorphenyl)-pyrrolidin-2,5-dion,
3-[N-(2-Methoxyäthoxy)-carbonylamino]-1-(3,5-dichlorphenyl)-pyrrolidin-2,5-dion,
3-(N-Isopropylthio-carbonylamino)-1-(3,5-dichlorphenyl)-pyrrolidin-2,5-dion und
3-(N-(Äthylthio-carbonylamino)-1-(3,5-dichlorphenyl)-pyrrolidin-2,5-dion.

Verbindungen der Formel I können, wir nachfolgend im einzelnen aufgeführt, hergestellt werden. In den Formeln II, III, IV und V haben die Substituenten $R_1$, $R_2$ und $R_3$ die unter Formel I angegebenen Werte, $R_6$ steht für $-NH-CO-R_4$ oder für $-NHR_7$, wobei $R_4$ die unter Formel I angegebene Bedeutung hat und $R_7$ für eine in der Peptid-Chemie übliche Schutzgruppe steht wie z.B. für Benzoxycarbonyl, tert.-Butoxycarbonyl usw. Hal steht für Halogen, z.B. für Fluor, Chlor, Brom oder Jod, insbesondere für Chlor oder Brom.

Die Verbindungen der Formel I werden erfindungsgemäss hergestellt durch Kondensations-Reaktion eines substituierten Anilins II mit einem Asparaginsäure-Derivat III

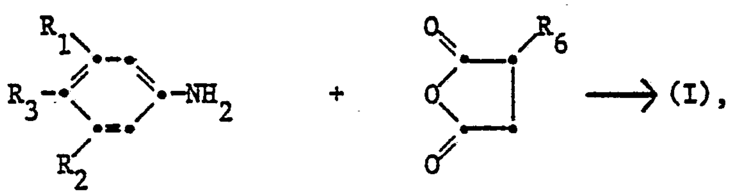


(II) (III)

worin $R_6$ für $-NH-CO-R_4$ oder $-NHR_7$ steht und $R_1$, $R_2$, $R_3$ und $R_4$ die unter Formel I angegebenen Bedeutungen haben, während $R_7$ für eine in der Peptid-Chemie übliche Schutzgruppe steht und, falls $R_6$ für $-NHR_7$ steht, die Reaktion über ein Zwischenprodukt der Formel IV

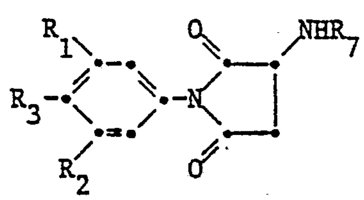

(IV)

verläuft, wobei die Schutzgruppe in an sich bekannter Weise abgespalten wird und die Aminogruppe mit einem für eine N-Acylierung reaktionsfähigen Carbonsäure-[Derivat] zu dem Endprodukt der Formel I reagiert.

Die Mikrobizide der Formel I sind luftstabile, in Wasser nahezu unlösliche, in den meisten üblichen organischen Lösungsmitteln lösliche Substanzen, die durch starke Laugen und Säuren zerstört werden.

Die Kondensationsreaktion (II + III → I) wird zweckmässigerweise in einem reaktionsinerten Lösungsmittel unter Normaldruck durchgeführt.

Beispiele solcher Lösungsmittel sind:
Aromatische und aliphatische Kohlenwasserstoffe wie Benzol, Toluol, Xylole, Petroläther, Ligroin oder Cyclohexan; halogenierte Kohlen-Wasserstoffe wie Chlorbenzol, Methylenchlorid, Aethylenchlorid, Chloroform, Tetrachlorkohlenstoff oder Tetrachloräthylen; Aether und ätherartige Verbindungen wie Diäthyläther, Diisopropyläther, t-Butylmethyläther, Dimethoxyäthan, Dioxan, Tetrahydrofuran oder Anisol; Ester wie Aethykacetat, Propylacetat oder Butylacetat; oder Verbindungen wie Dimethylsulfoxid, Dimethylformamid und Gemische solcher Lösungsmittel untereinander.

Bei dieser Reaktion kann die Gegenwart eines wasserentziehenden Mittels vorteilhaft sein. Man kann hierzu Carbonsäureanhydride wie Essigsäureanhydrid, Propionsäureanhydrid oder Verbindungen wie Acetylchlorid oder Thionylchlorid einsetzen. Diese Kondensationsmittel können auch als Lösungsmittel fungieren. Als wasserentziehende kommen auch Dicyclohexylcarbodiimid und Molekularsiebe in Frage.

Zur Beschleunigung dieser Kondensations-Reaktion arbeitet man beispielsweise bei Temperaturen von -10° bis +150°C, bevorzugt bei 0° bis +130°C bzw. am Siedepunkt des Lösungsmittels bzw. des Lösungsmittelgemisches. Als Katalysator kann Natriumacetat zugesetzt werden.

Als Amino-Schutzgruppen können alle in der Peptid-Chemie üblichen Amino-Schutzgruppen verwendet werden, wie sie in den entsprechenden Nachschlagewerken z.B. in Houben-Weyl: Methoden der organischen

Chemie; 4. Auflage, Band 15/I, E. Wünsch (Herausgeber): Synthese von Peptiden. (Georg Thieme Verlag, Stuttgart; 1974) zusammenfassend beschrieben werden. Bevorzugt sind acidolytisch abspaltbare Gruppen, wie in erster Linie die tert-Butoxycarbonylgruppe und analoge Gruppen, z.B. die tert-Amyloxycarbonyl-, Isopropyloxycarbonyl-, Diisopropylmethoxycarbonyl-, Allyloxycarbonyl-, Cyclopentyloxycarbonyl-, Cyclohexyloxycarbonyl-, d-Isobornyloxycarbonyl- und Adamantyloxycarbonylgruppe, sowie auch Gruppen des Aralkyl-Typs, wie Benzhydryl und Triphenylmethyl (Trityl), oder bestimmte Aralkoxycarbonyl-Gruppen des 2-(p-Biphenyl)-2-propyloxycarbonyl-Typs, die in den US-Patentschriften 3 875 207 und 3 944 590 beschrieben sind.

Ferner kann man aber auch reduktiv oder unter milden Bedingungen basisch abspaltbare Amino-Schutzgruppen verwenden, z.B. insbesondere die Benzyloxycarbonyl-Gruppe und Benzyloxycarbonyl-Gruppen, die im aromatischen Teil durch Halogenatome, Nitrogruppen, Niederalkoxygruppen und/oder Niederalkylreste substituiert sind, wie die p-Chlor- und p-Brombenzyloxycarbonyl-, p-Nitrobenzyloxycarbonyl-, p-Methoxybenzyloxycarbonyl-, p-Tolyloxycarbonyl-Gruppe, oder aber die Isonicotinyloxycarbonylgruppe, weiter auch Acylgruppen, wie p-Toluolsulfonyl, Benzolsulfenyl, o-Nitrobenzolsulfenyl bzw. auch Formyl, Trifluoracetyl oder Phthaloyl.

Eine besonders vorteilhafte Amino-Schutzgruppe ist eine Aethoxycarbonylgruppe, die in β-Stellung eine mit drei Kohlenwasserstoffresten substituierte Silylgruppe, wie Triphenylsilyl-, Dimethylbutylsilyl- oder vor allem Trimethylsilylgruppe, trägt. Eine solche β-Trihydrocarbylsilyl)-äthoxycarbonylgruppe, wie eine β-(Triniederalkylsilyl)-äthoxycarbonyl- z.B. insbesondere die β-(Trimethylsilyl)-äthoxycarbonylgruppe, bildet mit der zu schützenden Aminogruppe eine entsprechende β-Trihydrocarbylsilyl-äthoxycarbonylaminogruppe (z.B. die β-Trimethylsilyläthoxycarbonylaminogruppe) welche gegen die Bedingungen der sauren Hydrolyse und der Hydrogenolyse beständig ist, aber unter ganz spezifischen, sehr milden Bedingungen sich durch Einwirkung von Fluoridionen abspalten lässt.

Die Abspaltung der Schutzgruppe erfolgt in der allgemein bekannten Weise; die saure Hydrolyse (Acidolyse) wird z.B. mittels Trifluoressigsäure, Bromwasserstoffsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten.

Die anschliessende N-Acylierung wird zweckmässigerweise in den üblichen reaktionsinerten organischen Lösungsmitteln, bevorzugt unter milden Bedingungen (-10° bis +20°C) durchgeführt. Als Acylierungsmittel kommen reaktionsfähige Carbonsäure-[Derivate], insbesondere Säurehalogenide (z.B. Säurechloride und Säurebromide),-Ester und-Anhydride in Betracht. Bei dieser Reaktion ist es vorteilhaft die freiwerdende Säure durch ein geeignetes Bindemittel abzufangen. Hierzu eignen sich z.B. organische Basen wie Trialkylamine (Trimethylamin, Triäthylamin usw.), Pyridin und Pyridinbasen oder anorganische Basen wie Oxide, Hydroxide, Hydrogencarbonate, Carbonate oder Hydride von Alkali- und Erdalkalimetallen. Es ist zweckmässig die Umsetzung in Gegenwart von 2 Aequivalenten des Bindemittels (bezogen auf Produkt I) durchzuführen; als Katalysator lassen sich hierbei 4-Dialkylaminopyridine verwenden.

Substituierte Aniline der Formel II sind allgemein bekannt. Asparaginsäure-Derivate der Formel III sind grösstenteils bekannt und werden nach an sich bekannten Methoden aus der zugrundeliegenden

freien Asparaginsäure z.B. durch Acylierung der Säure und anschliessende intramolekulare Kondensation hergestellt. [Vgl. Houben-Weyl, Bd. 15/2, Seite 219 f.]. Die Verbindungen der Formel III sind auch durch Umsetzung von Asparaginsäure mit einem entsprechenden Carbonsäureanhydrid zugänglich. [Vgl. J. Med. Chem. 16, 163 (1973)].

Nach einer Variante des obigen Verfahrens werden die Verbindungen der Formel I, in denen $R_4$ einen Rest $-XR_5$ bedeutet, erhalten, indem man ein Asparaginsäurederivat V in Gegenwart von zwei Aequivalenten eines säurebindenden Mittels mit Phosgen umsetzt und das entstehende Carbamoylchlorid VI in Gegenwart eines Aequivalents eines säurebindenden Mittels mit einem Alkohol oder Thioalkohol VII verestert.

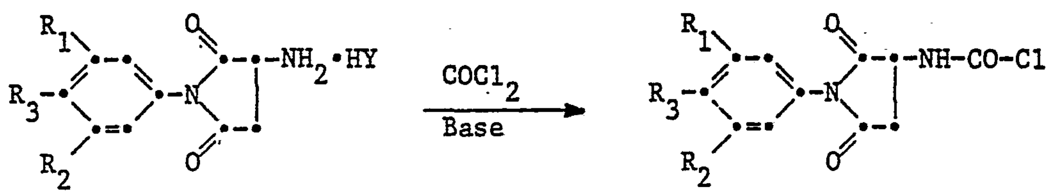


(V) (VI)

$HX-R_5$ (VII)
(I)
Base

In den Formeln V bis VII haben $R_1$ bis $R_3$ und X die unter Formel I angegebene Bedeutung. Y steht für Halogen, insbesondere Chlor oder Brom.

Die Chlorcarbonylierungsreaktion (V ⟶ VI) sowie die Veresterung (VI + VII ⟶ I) werden zweckmässigerweise in gegenüber den Reaktionspartnern inerten Lösungsmitteln durchgeführt. Dafür kommen die gleichen Lösungsmittel in Frage, wie sie für die obige Kondensationsreaktion angegeben werden.

Beispiele für säurebindende Mittel sind organische Basen wie Triäthylamin, Diäthylamin, Pyridin, 4-Dimethylaminopyridin oder anorganische Basen wie Natrium- und Kaliumhydroxid, Natrium- und Kaliumcarbonat, Natrium- und Kaliumhydrogencarbonat, Magnesium und Calciumoxid.

Die Chlorcarbonylierung wird bei Temperaturen von -50°C bis +50°C ausgeführt, vorzugsweise bei -40° bis -20°.

Die anschliessende Veresterung des Carbamoylchlorids zu Verbindungen der Formel I wird zwischen -20°C und den Siedepunkt des Reaktionsgemisches, vorzugsweise zwischen -10°C und +30°C durchgeführt.

Die Ausgangsverbindungen V werden durch Abspalten der Schutzgruppe $R_7$ mit Halogenwasserstoffsäure aus dem Zwischenprodukt IV des Hauptverfahrens direkt als Hydrogenhalogenide erhalten.

Das Herstellungsverfahren ist in allen beschriebenen Varianten ein Teil der Erfindung.

Die Verbindungen der Formel I

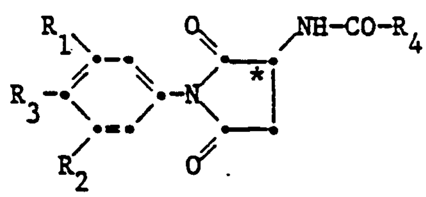

(I)

besitzen im Pyrrolidin Ring in Nachbarstellung zur Seitenkette ein asymmetrisches Kohlenstoffatom *C und können auf übliche Art als reine optische Antipoden von I synthetisiert werden, z.B. indem man bei der Herstellung von einer enantiomeren Form der Asparaginsäure ausgeht. Je nach Substitution treten weitere Asymmetrie-Zentren auf. Verbindungen der Formel I bilden auch verschiedene Diastereomere.

Die verschiedenen Isomeren besitzen unterschiedliche mikrobizide Wirkung. Sofern keine gezielte Synthese zur Isolierung reiner Isomeren durchgeführt wird, fällt ein Produkt der Formel I stets als Gemisch dieser Isomeren an.

Es wurde überraschend gefunden, dass Verbindungen der Formel I ein für praktische Bedürfnisse sehr günstiges Mikrobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative und präventive Wirkung zum Schutz von Kulturpflanzen ohne diese durch unerwünschte Nebenwirkungen zu beeinflussen. Kulturpflanzen seien im Rahmen vorliegender Erfindung beispielsweise: Getreide: (Weizen, Gerste, Roggen, Hafer, Reis);
Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute), Citrusfrüchte: Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Wirkstoffe der Formel I sind unter anderem gegen folgende phytopathogene Pilze wirksam: z.B. gegen die zur Familie der Ascomycetes gehörenden Erysiphaceae sowie gegen Fungi imperfecti wie Botrytis und Monilinia.

Sie können ferner als Beizmittel zur Behandlung von Saatgut (Früchte, Knollen, Körner) und Pflanzenstecklingen zum Schutz vor Pilzinfektionen sowie gegen im Erdboden auftretende Mikroorganismen eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Zur Bekämpfung dieser Mikroorganismen können die Verbindungen der Formel I für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Wirkstoffe der Formel I können auch im Gemisch mit z.B. pestiziden oder pflanzenwuchsverbessernden Präparaten verwendet werden.

Der Gehalt an Wirkstoff liegt dabei in handelsfähigen Mitteln im allgemeinen zwischen 0,0001 und 90 %.

In den nachfolgenden Beispielen sind Temperaturen in Celsiusgraden angegeben, Teile bedeuten Gewichtsteile. Prozentangaben beziehen sich stets auf das Gewicht.

<u>Herstellungsbeispiele</u>

<u>Beispiel 1:</u> Herstellung von

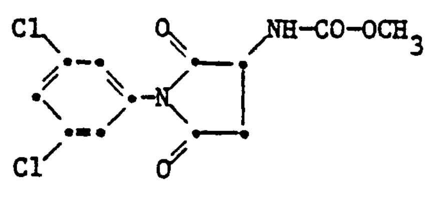

(2.1)

<u>3-(N-Methoxycarbonylamino)-1-(3,5-dichlorphenyl)-pyrrolidin-2,5-dion</u>

12,9 g N-Methoxycarbonylasparaginsäureanhydrid werden in 100 ml Tetrahydrofuran gelöst und mit einer Lösung von 12 g 3,5-Dichloranilin in 50 ml Tetrahydrofuran versetzt. Die Lösung wird 4 Stunden bei 50° gerührt und anschliessend im Vakuum eingeengt. Der Rückstand wird in 80 ml Essigsäureanhydrid gelöst, mit 2,5 g Natriumacetat versetzt und 1,5 Stunden bei 80° gerührt. Nach Abkühlen auf Raumtemperatur wird das Reaktionsgemisch auf Wasser gegossen und 0,5 Stunden heftig gerührt. Das Gemisch wird mit Essigester extrahiert. Die Extrakte nacheinander mit gesättigter Natriumhydrogencarbonat- und gesättigter Natriumchloridlösung gewaschen, über Natriumsulfat getrocknet, filtriert und eingeengt. Nach Umkristallisation des Rückstandes erhält man farblose Kristalle obiger Verbindung, die einen Smp. von 136°-138° aufweisen.

Beispiel 2: a) Herstellung eines Zwischenproduktes

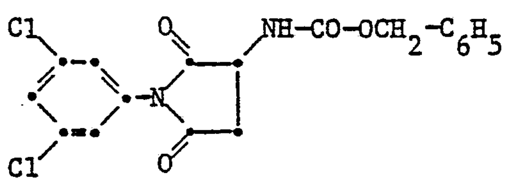

3-(N-Benzyloxycarbonylamino)-1-(3,5-dichlorphenyl)-pyrrolidin-2,5-dion

Zu einer Lösung von 44 g N-Benzyloxycarbonylasparaginsäureanhydrid in 200 ml Tetrahydrofuran lässt man eine Lösung von 32 g 3,5-Dichloranilin in 100 ml Tetrahydrofuran tropfen. Nach 16 stündigem Stehen bei Raumtemperatur wird die Lösung eingedampft, der Rückstand in 200 ml Essigsäureanhydrid aufgenommen, mit 4 g Natriumacetat versetzt und eine Stunden bei 80° gerührt. Anschliessend wird das Reaktionsgemisch auf Raumtemperatur abgekühlt, auf Wasser gegossen und 30 Minuten kräftig gerührt. Der kristalline Rückstand wird abfiltriert, mit wenig kaltem Wasser verrieben, nochmals filtriert, gut mit Wasser gewaschen und im Vakuum getrocknet. Die farblosen Kristalle schmelzen bei 112-113°.

b) Herstellung eines weiteren Zwischenproduktes

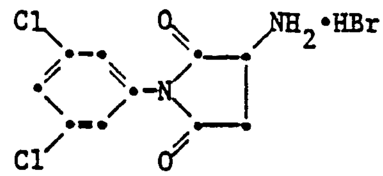

3-Amino-1-(3,5-dichlorphenyl)-pyrrolidin-2,5-dion-hydrobromid

85 g 3-(N-Benzyloxycarbonylamino)-1-(3,5-dichlorphenyl)-pyrrolidin-2,5-dion werden mit 200 ml einer mit Bromwasserstoff gesättigten Eisessiglösung vermischt und 1,5 Stunden bei Raumtemperatur gerührt. Anschliessend wird 1 Liter Diäthyläther hinzugegeben und die ausfallenden Kristalle rasch abfiltriert, mit Diäthyläther gewaschen und über Kaliumhydroxid im Vakuum getrocknet. Das Hydrobromid fällt in Form farbloser Kristalle an, die bei 278°-280° unter Zersetzung schmelzen.

c) Herstellung eines Endproduktes

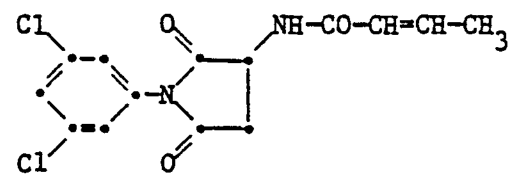

(1.25)

3-(N-Crotylamino)-1-(3,5-dichlorphenyl)-pyrrolidin-2,5-dion

Zu 13,6 g 3-Amino-1-(3,5-dichlorphenyl)-pyrrolidin-2,5-dion-hydrobromid in 100 ml Tetrahydrofuran werden bei 0° 12 ml Triäthylamin gegeben. Anschliessend lässt man 6 ml Crotonsäurechlorid zutropfen und rührt das Reaktionsgemisch zuerst 5 Stunden bei 0°, anschliessend weitere 12 Stunden bei Raumtemperatur. Die Lösung wird dann filtriert und das Filtrat eingeengt und säulenchromatographisch [Kieselgel-Säule / Dichlormethan-Petroläther (85:15)] gereinigt. Nach Entfernen des Lösungsmittels wird der Rückstand aus Aethylacetat-Petroläther umkristallisiert. Die farblosen Kristalle schmelzen bei 107-110°.

Beispiel 3: Herstellung von

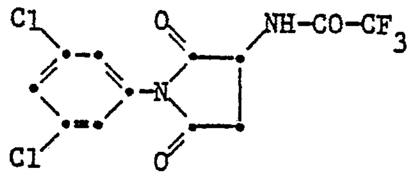

(1.11)

3-(N-Trifluoracetylamin)-1-(3,5-dichlorphenyl)-pyrrolidin-2,5-dion

Zu einer Lösung von 40 g Trifluoracetylasparaginsäureanhydrid in 100 ml Tetrahydrofuran lässt man bei Raumtemperatur eine Lösung von 30 g 3,5-Dichloranilin in 100 ml Tetrahydrofuran zutropfen. Das Reaktionsgemisch wird 16 Stunden bei 50° gerührt und anschliessend eingedampft. Der kristalline Rückstand wird in 150 ml Essigsäureanhydrid zusammen mit 3 g Natriumacetat 2 Stunden bei 50° gerührt. Dann wird die Lösung auf Wasser gegossen und 1,5-Stunden gerührt. Der anfallende Niederschlag wird abfiltriert, mit Wasser gewaschen, getrocknet und aus Aetylacetat-Petroläther umkristallisiert. Man erhält farblose Kristalle mit Smp. 147-148°.

Beispiel 4:

a) Herstellung eines Zwischenproduktes

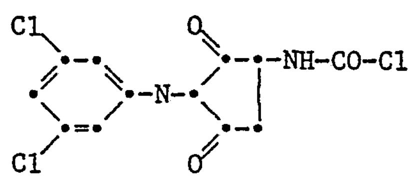

3-(N-Chlorcarbonylamino)-1-(3,5-dichlorphenyl)-pyrrolidin-2,5-dion

In 104 ml einer 20%igen Lösung von Phosgen in Toluol werden nach Verdünnung mit 200 ml Tetrahydrofuran bei -20°C 34 g 3-Amino-1--(3,5-dichlorphenyl)-pyrrolidin-2,5-dion-hydrobromid (erhalten nach Beispiel 2b) eingetragen. Nach anschliessendem Zutropfenlassen von 27,9 g Triäthylamin in 100 ml Tetrahydrofuran bei -37°C bis -28°C wird

die Reaktionsmischung zur Vervollständigung der Reaktion für weitere 12 Stunden bei -33°C gerührt. Nach Abfiltrieren des Niederschlages bei -20°C und Einengen der Lösung wird das entstandene 3-(N-Chlorcarbonyl-amino)-1-(3,5-dichlorphenyl)-pyrrolidin-2,5-dion direkt für die Folgereaktion eingesetzt.

b) Herstellung von

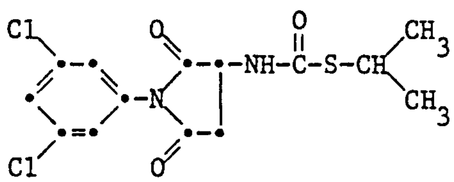

(2.18)

3-(N-Isopropylthio-carbonylamino)-1-(3,5-dichlorphenyl)-pyrrolidin--2,5-dion

Zur auf -5°C gekühlten Lösung von 0,3 g 4-Dimethylaminopyridin, 30,7 ml Triäthylamin und 20,6 ml Isopropylmercaptan in 200 ml Tetrahydrofuran lässt man langsam eine Lösung des unter a) erhaltenen 3-(N-Chlorcarbonylamino)-1-(3,5-dichlorphenyl)-pyrrolidin-2,5-dions in 100 ml Tetrahydrofuran zutropfen. Nachdem das Reaktionsgemisch für 12 Stunden bei Raumtemperatur gerührt worden ist, wird der entstandene Niederschlag abfiltriert, das Filtrat eingeengt und der Rückstand aus Isopropanol kristallisiert. Das farblose Endprodukt kristallisiert mit einem Mol-Aequivalent Isopropanol, Smp. 161°-163°C.

Auf analoge Weise werden folgende Endprodukte der Formel I hergestellt:

Tabelle I    Verbindungen der Formel I

| Verb. Nr. | $R_1$ | $R_2$ | $R_3$ | $R_4$ | physik. Konst. |
|---|---|---|---|---|---|
| 1.1 | Cl | Cl | H | $-CH_3$ | Smp. 140-142°C |
| 1.2 | Cl | Cl | H | $-CH_2Cl$ | Smp. 140-142°C |
| 1.3 | Cl | Cl | H | $-CH_2Br$ | Smp. 143-145°C |
| 1.4 | Cl | Cl | H | $-CH_2J$ | Smp. 169-172°C |
| 1.5 | Cl | Cl | H | $-CHCl_2$ | |
| 1.6 | Cl | Cl | H | $-CCl_3$ | |
| 1.7 | Br | Br | H | $-CH_3$ | |
| 1.8 | Br | Br | H | $-CH_2Br$ | |
| 1.9 | Cl | Cl | F | $-CH_3$ | |
| 1.10 | Br | Br | F | $-CF_3$ | |
| 1.11 | Cl | Cl | H | $-CF_3$ | Smp. 147-148°C |
| 1.12 | Cl | Cl | H | $-C_2H_5$ | |
| 1.13 | Cl | Cl | H | $-C_3H_7-n$ | Smp. 127-129°C |

Tabelle I Fortsetzung

| Verb. Nr. | $R_1$ | $R_2$ | $R_3$ | $R_4$ | physik. Konst. |
|---|---|---|---|---|---|
| 1.14 | Cl | Cl | H | $-C_7H_{15}-n$ | |
| 1.15 | Cl | Cl | H | $-C_2Cl_5$ | |
| 1.16 | Br | Br | H | $-C_4H_9-n$ | |
| 1.17 | Br | Br | F | $-C_2H_5$ | |
| 1.18 | Cl | Cl | H | Cyclopropyl | Smp. 162-164°C |
| 1.19 | Cl | Cl | F | Cyclopropyl | |
| 1.20 | Cl | Cl | H | Cyclohexyl | Smp. 175-176°C |
| 1.21 | Cl | Cl | H | $-CH=CH_2$ | Smp. 153-155°C |
| 1.22 | Cl | Cl | H | $-CCl=CCl_2$ | Smp. 136-138°C |
| 1.23 | Br | Br | H | $-CH=CH_2$ | |
| 1.24 | Cl | Cl | F | $-CH=CH_2$ | |
| 1.25 | Cl | Cl | H | $-CH=CH-CH_3$ | Smp. 107-110°C |
| 1.26 | Cl | Cl | H | $-CH=C(CH_3)_2$ | amorph |
| 1.27 | Cl | Cl | H | $-(CH=CH)_2CH_3$ | Smp. 184-186°C |
| 1.28 | Br | Br | H | $-CH=C(CH_3)_2$ | |
| 1.29 | Cl | Cl | F | $-CH=C(CH_3)_2$ | |
| 1.30 | Cl | Cl | H | $-C(CH_3)=CH-CH_3$ | Smp. 47-48°C |
| 1.31 | Cl | Cl | F | $-C(CH_3)=CH_2$ | |
| 1.32 | $NO_2$ | Cl | H | $-CH=CH-C_6H_5$ | |
| 1.33 | Cl | Cl | F | $-C(CH_3)=CH_2$ | |
| 1.34 | Cl | Cl | F | $-C(CH_3)=C(CH_3)_2$ | |
| 1.35 | Cl | Cl | H | $-CH=C(C_2H_5)_2$ | |
| 1.36 | Cl | Cl | H | $-C(C_2H_5)=CH_2$ | |
| 1.37 | Cl | Cl | H | Furyl | Harz |
| 1.38 | Cl | Cl | F | Tetrahydro-furyl | |
| 1.39 | Cl | Cl | H | Pyrrolyl | |
| 1.40 | $NO_2$ | Cl | H | $-C_6H_5$ | |
| 1.41 | Cl | Cl | F | $-C_6H_5$ | |
| 1.42 | Cl | Cl | H | $-C_6H_3Cl_2$ (3,4) | |

Tabelle I Fortsetzung

| Verb. Nr. | $R_1$ | $R_2$ | $R_3$ | $R_4$ | phys. Konst. |
|---|---|---|---|---|---|
| 1.43 | Cl | Cl | F | $-C_6H_3Cl_2(2,5)$ | |
| 1.44 | Cl | Cl | H | $-(CH_2)_8CH_3$ | Smp. 85-88°C |
| 1.45 | Cl | Cl | H | $-CH_2OC_3H_7$-n | |
| 1.46 | Cl | Cl | F | $-CH_2OC_2H_5$ | |
| 1.47 | Br | Br | H | $-CH_2OC_3H_7$-i | |
| 1.48 | Cl | Cl | H | $-CH_2OCH_3$ | Smp. 157-172°C |
| 1.49 | Cl | Cl | H | $-C_6H_4CH_3(2)$ | |
| 1.50 | Cl | Cl | H | $-C_6H_3(CH_3)_2(3,4)$ | |
| 1.51 | $NO_2$ | Cl | H | $-CH=CH_2$ | |
| 1.52 | $NO_2$ | Cl | H | $-C_3H_7$-n | |
| 1.53 | Cl | Cl | H | $-CH=CH-C_6H_5$ | Smp. 174-175°C |
| 1.54 | Cl | Cl | H | $-C(CH_3)=CH_2$ | Smp. 125-128°C |
| 1.55 | Cl | Cl | H | $-C_3H_7$-i | Smp. 160-161°C |
| 1.56 | Cl | Cl | H | $-C(CH_3)_3$ | Smp. 185-188°C |

Tabelle II: Verbindungen der Formel I

| Verb. Nr. | $R_1$ | $R_2$ | $R_3$ | $-X-R_5$ | physik. Konst. |
|---|---|---|---|---|---|
| 2.1 | Cl | Cl | H | $-OCH_3$ | Smp. 136-138°C |
| 2.2 | Br | Br | H | $-OCH_3$ | |
| 2.3 | Cl | Cl | H | $-S-CH_2-CH=CH_2$ | wachsartig |
| 2.4 | Cl | Cl | H | $-SCH_3$(x i-$C_3H_7OH$) | Smp. 73-76°C |
| 2.5 | Cl | Cl | H | $-OC_2H_5$ | Smp. 67-69°C |
| 2.6 | Cl | Cl | H | $-SC_2H_5$ | Smp. 118-123°C |
| 2.7 | Cl | Cl | F | $-OCH_2-CH_3$ | |
| 2.8 | Cl | Cl | H | $-OCH_2-C_3H_7-i$ | Smp. 152-155°C |
| 2.9 | Br | Br | H | $-OCH_2-C_6H_5$ | - |
| 2.10 | $NO_2$ | Cl | H | $-OCH_3$ | |
| 2.11 | Cl | Cl | H | $-OCH_2-C_6H_5$ | Smp. 112-113°C |
| 2.12 | Cl | Cl | H | $-O-CH_2-CH_2-OCH_3$ | Smp. 109-110°C |
| 2.13 | $NO_2$ | Cl | H | $-OC_3H_7-n$ | |
| 2.14 | Cl | Cl | H | $-OCH_2CH=CH_2$ | Smp. 56-58°C |
| 2.15 | Cl | Cl | H | $-O-CH_2-CH_2-Br$ | Smp. 131-134°C |
| 2.16 | Br | Br | H | $-O-CH_2-CCl_3$ | |
| 2.17 | Cl | Cl | H | $-O-CH_2-CCl_3$ | sintert ab 80°C |
| 2.18 | Cl | Cl | H | $-S-C_3H_7-i$(x i-$C_3H_7OH$) | Smp. 161-163°C |
| 2.19 | Cl | Cl | H | $-OC_6H_5$ | |
| 2.20 | Br | Br | H | $-OC_6H_5$ | |
| 2.21 | Cl | Cl | H | $-S-C_4H_9-n$ | Smp. 99-104°C |
| 2.22 | Cl | Cl | H | $-OC_6H_3(CH_3)_2$(2,6) | |
| 2.23 | Cl | Cl | H | $-SC_6H_5$ | |
| 2.24 | Cl | Cl | F | $-SCH_2C_6H_5$ | |
| 2.25 | Cl | Cl | H | $-OCH_2C_6H_3(CH_3)_2$(2,6) | |
| 2.26 | Cl | Cl | F | $-SC_3H_7-i$ | |
| 2.27 | Br | Br | H | $-SCH_3$ | |
| 2.28 | Cl | Cl | H | $-S-C(CH_3)_3$ | Smp. 174-182°C |

Zur Applikation können die Verbindungen der Formel I in den folgenden Aufarbeitungsformen vorliegen:

Formulierungsbeispiele

Beispiel 4:

Feste Aufarbeitungsformen:

Stäube- und Streumittel enthalten im allgemeinen bis zu 10% des Wirkstoffs. Ein 5%-iges Stäubemittel kann beispielsweise aus 5 Teilen des Wirkstoffs und 95 Teilen eines Zuschlagsstoffes wie Talkum bestehen oder aus 2 Teilen Wirkstoff, 1 Teil hochdisperser Kieselsäure und 97 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

Granulate wie Umhüllungsgranulate, Imprägniergranulate, Homogengranulate und Pellets [=Körner] enthalten üblicherweise 1 bis 80 % des Wirkstoffs. So kann sich ein 5 %-iges Granulat z.B. aus 5 Teilen des Wirkstoffs, 0,25 Teilen epoxydierten Pflanzenöles, 0,25 Teilen Cetylpolyglykoläther, 3,50 Teilen Polyäthylenglykol und 91 Teilen Kaolin (bevorzugt Korngrösse 0,3-0,8 mm) zusammensetzen. Man kann bei der Herstellung des Granulates wie folgt vorgehen:

Die Aktivsubstanz wird mit epoxydiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 5:

Flüssige Aufarbeitungsformen:

Man unterscheidet im allgemeinen zwischen Wirkstoffkenzentraten, die in Wasser dispergierbar oder löslich sind und Aerosolen. Zu den in Wasser dispergierbaren Wirkstoffkonzentraten zählen z.B. Spritzpulver (wettable powders) und Pasten, die üblicherweise in den Handelspackungen 25-90% und in gebrauchsfertigen Lösungen 0,01 bis 15% des Wirkstoffs enthalten. Emulsionskonzentrate enthalten 10 bis 50% und Lösungskonzentrate enthalten in der gebrauchsfertigen Lösung 0,001 bis 20% Aktivsubstanz. So kann ein 70%-iges Spritzpulver z.B. aus 70 Teilen des Wirkstoffs, 5 Teilen Natriumdibutylnaphthylsulfonat, dazu 3 Teilen Naphthalinsulfonsäuren - Phenolsulfonsäuren - Formaldehyd-Kondensat (im Mischungsverhältnis 3:2:1), 10 Teilen Kaolin und 12 Teilen Kreide z.B. Champagne-Kreide zusammengesetzt sein. Ein 40%-iges Spritzpulver kann z.B. aus folgenden Stoffen bestehen: 40 Teile Wirkstoff, 5 Teile Natrium-Ligninsulfonat, 1 Teil Natrium-Dibutylnaphthylsulfonat und 54 Teile Kieselsäure. Die Herstellung eines 25%-igen Spritzpulvers kann auf unterschiedliche Art erfolgen. So kann dieses sich z.B. zusammensetzen aus: 25 Teilen der Aktivsubstanz, 4,5 Teilen Calcium-Ligninsulfonat, 1,9 Teilen Kreide, z.B. Champagne-Kreide/Hydroxyäthylencellulose-Gemisch (1:1), 1,5 Teilen Natrium-Dibutylnaphthylsulfonat, 19,5 Teilen Kieselsäure, 19,5 Teilen Champagne-Kreide und 28,1 Teilen Kaolin. Ein 25%-iges Spritzpulver kann z.B. auch bestehen aus 25 Teilen Wirkstoff, 2,5 Teilen Isooctylphenoxypolyoxyäthylen-äthanol, 1,7 Teilen Champagne-Kreide/Hydroxyäthylcellulosegemisch (1:1), 8,3 Teilen Natriumsilikat, 16,5 Teilen Kieselgur und 46 Teilen Kaolin. Ein 10%-iges Spritzpulver lässt sich z.B. herstellen aus 10 Teilen des Wirkstoffes, 3 Teilen eine Gemisches aus Natriumsalzen von gesättigten Fettalkoholsulfonaten, 5 Teilen Naphthalinsulfonsäure/Formaldehyd-Kondensat und 82 Teilen Kaolin. Andere Spritzpulver können Gemische darstellen aus 5 bis 30% der Aktivsubstanz zusammen mit 5 Teilen eines aufsaugenden Trägermaterials

wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykoläthers. Ein 25%-iges Emulsions-Konzentrat kann z.B. folgende emulgierbare Stoffe enthalten: 25 Teile des Wirkstoffs, 2,5 Teile epoxidiertes Pflanzenöl, 10 Teile eines Alkylarylsulfonat-Fettalkoholpolyglykoläther-Gemisches, 5 Teile Dimethylformamid und 57,5 Teile Xylol.

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen. Solche Mittel gehören ebenfalls zur Erfindung.

Mittel, die wie oben beschrieben, als Wirkkomponente eine Verbindung der Formel I enthalten, beispielsweise Verbindung Nr. 1.1, 1.2, 1.3, 1.4, 1.11, 1.13, 1.18, 1.20, 1.21, 1.22, 1.25, 1.26, 1.27, 1.30, 1.37, 1.42, 1.44, 1.48, 1.53, 1.54, 1.55, 1.56, 2.1, 2.3, 2.4, 2.5, 2.6, 2.8, 2.11, 2.12, 2.14, 2.15, 2.17, 2.18, 2.21 und 2.28 lassen sich mit gutem Erfolg gegen schädliche Mikroorganismen einsetzen, insbesondere gegen blatt-, boden- und samenbürtige Pilze, wenn sie beispielsweise in Form eines Spritzmittels appliziert werden.

Mit gleichgutem oder ähnlichem Erfolg lassen sich auch Mittel einsetzen, die als Wirkstoff eine der übrigen Substanzen aus den Tabellen I und II enthalten.

## Biologische Beispiele:

### Beispiel 6: Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellt Spritzbrühe gegossen (0.006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Bei der Behandlung von Gerstenpflanzen gegen Erysiphe-Pilze mit Aktivsubstanzen der Formel I, wie z.B. Verbindung Nr. 1.4, 1.11, 1.18, 1.54, 2.1 und 2.5 wurde der Befall der behandelten Pflanzen im Vergleich mit Kontrollpflanzen (100% Befall) auf weniger als 10% zurückgedrängt. Darüberhinaus zeigte Verbindung Nr. 1.4 zusätzlich eine systemische Wirkung.

Beispiel 7: Wirkung gegen Botrytis cinerea auf Bohnen

Residual-protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach eine Inkubation der infizierten Pflanzen während 3 Tagen bei 95-100% relativer Luftfeuchtigkeit und 21°C erfolgte die Beurteilung des Pilzbefalls.

Bohnenpflanzen, die mit einer Spritzbrühe behandelt worden sind, die als Aktivsubstanz eine der Verbindungen Nr. 1.2, 1.3, 1.4, 1.18, 1.25, 1.26, 1.27, 2.1, 2.3, 2.5, 2.8, 2.14, 2.15 oder 2.18 enthielten, zeigten im Gegensatz zu unbehandelten Kontrollpflanzen (100% Befall) weniger als 5% Befall.

Beispiel 8: Wirkung gegen Monilinia auf Pflanzenblüten

Einzelne voll erblühte Pflaumenzweige werden mit einer aus einem Emulsionskonzentrat hergestellten Spritzbrühe (enthaltend 0,025% Aktivsubstanz) besprüht. Einige Stunden später werden die Blütenstände abgeschnitten, mit dem Stiel in den nassen Stand von Plastikschalen gesteckt und mit einer Sporensuspension inokuliert. Die Schalen werden dann lose mit transparenter Plastikfolie bedeckt und 2 Tage bei Raumtemperatur gehalten. Mit der Zahl von befallenen Blüten wird das Ausmass des Krankheitsbefalls bestimmt, wobei je 40 Blüten pro Wirkstoff verwendet werden. Die Verbindungen Nr. 1.26, 1.27 und 2.1 bewirken eine Reduktion des Krankheitsbefalls auf unter 20%. Die Verbindung 2.1 verhütete den Befall vollständig.

Patentansprüche

1. Verbindungen der Formel I

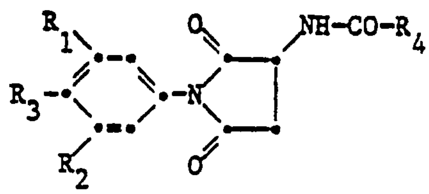

(I),

worin $R_1$ für Chlor, Brom oder Nitro, $R_2$ für Chlor oder Brom, $R_3$ für Wasserstoff oder Fluor steht, $R_4$ ein gegebenenfalls durch Halogen oder $C_1$-$C_3$-Alkoxy substituiertes $C_1$-$C_9$-Alkyl, ein gegebenenfalls durch Halogen oder Aryl substituiertes $C_2$-$C_7$-Alkenyl, $C_2$-$C_4$-Alkinyl, $C_3$-$C_6$-Cycloalkyl, ein gegebenenfalls durch Halogen, Nitro oder $C_1$-$C_4$-Alkyl substituiertes Phenyl, einen 5-gliedrigen Heterocyclus mit einem Heteroatom oder -X-$R_5$ bedeutet, wobei X für Sauerstoff oder Schwefel steht und $R_5$ ein gegebenenfalls durch Halogen oder $C_1$-$C_3$-Alkoxy substituiertes $C_1$-$C_6$-Alkyl oder substituiertes $C_3$-$C_6$-Alkenyl, ein gegebenenfalls durch Halogen, Nitro, $C_1$-$C_3$-Alkyl oder $C_1$-$C_3$-Alkoxy substituiertes Phenyl oder ein gegebenenfalls durch Halogen oder Nitro substituiertes Benzyl bedeutet.

2. Verbindungen der Formel I nach Anspruch 1, worin $R_1$ und $R_2$ Chlor, $R_3$ Wasserstoff, $R_4$ gegebenenfalls durch Halogen substituiertes $C_1$-$C_4$-Alkyl, gegebenenfalls durch Halogen oder Aryl substituiertes $C_2$-$C_7$-Alkenyl, gegebenenfalls durch Halogen oder $C_1$-$C_2$-Alkyl substituiertes Phenyl, X Sauerstoff oder Schwefel und $R_5$ gegebenenfalls durch Halogen oder $C_1$-$C_3$-Alkoxy substituiertes $C_1$, $C_6$-Alkyl, $C_3$-$C_6$-Alkenyl oder gegebenenfalls durch Halogen oder $C_1$-$C_3$-Alkyl substituiertes Phenyl bedeuten.

3. Verbindungen der Formel I nach Anspruch 1, worin $R_1$ und $R_2$ für Chlor stehen und $R_4$ ein gegebenenfalls durch Halogen oder Aryl substituiertes $C_2$-$C_7$-Alkenyl bedeutet.

4. Verbindungen der Formel I nach Anspruch 1, worin $R_1$ und $R_2$ für Chlor stehen, $R_3$ Wasserstoff darstellt und $R_4$ für $-OR_5$ steht, wobei $R_5$ ein gegebenenfalls durch Halogen oder $C_1$-$C_3$-Alkoxy substituiertes $C_1$-$C_6$-Alkyl bedeutet.

5. Verbindungen der Formel I nach Anspruch 1, worin $R_1$ und $R_2$ für Chlor stehen, $R_3$ Wasserstoff bedeutet und $R_4$ für $-SR_5$ steht, wobei $R_5$ ein gegebenenfalls durch Halogen oder $C_1$-$C_3$-Alkoxy substituiertes $C_1$-$C_6$-Alkyl bedeutet.

6. 3-(N-Methoxycarbonylamino)-1-(3,5-dichlorphenyl)-pyrrolidin-3,5--dion nach Anspruch 1.

7. 3-(N-Äthoxycarbonylamino)-1-(3,5-dichlorphenyl)-pyrrolidin-2,5--dion nach Anspruch 1.

8. 3-(N-Trifluoracetylamino)-1-(3,5-dichlorphenyl)-pyrrolidin-2,5--dion nach Anspruch 1.

9. 3-[N-(2-Methyl-1-propenyl)-carbonylamino]-1-(3,5-dichlorphenyl)--pyrrolidin-2,5-dion nach Anspruch 1.

10. 3-[N-(1,3-Pentadienyl)-carbonylamino]-1-(3,5-dichlorphenyl)--pyrrolidin-2,5-dion nach Anspruch 1.

11. 3-(N-Cyclopropylcarbonylamino)-1-(3,5-dichlorphenyl)-pyrrolidin--2,5-dion nach Anspruch 1.

12. 3-(N-Isobutyloxycarbonylamino)-1-(3,5-dichlorphenyl)-pyrrolidin--2,5-dion nach Anspruch 1.

13. 3-[N-(2-Propenyloxy)-carbonylamino]-1-(3,5-dichlorphenyl)-pyrrolidin-2,5-dion nach Anspruch 1.

14. 3-[N-(2-Bromäthoxy)-carbonylamino]-1-(3,5-dichlorphenyl)-pyrrolidin-2,5-dion nach Anspruch 1.

15. 3-[N-(2-Methoxyäthoxy)-carbonylamino]-1-(3,5-dichlorphenyl)-pyrrolidin-2,5-dion nach Anspruch 1.

16. 3-(N-Isopropylthio-carbonylamino)-1-(3,5-dichlorphenyl)-pyrrolidin-2,5-dion nach Anspruch 1.

17. 3-(N-Äthylthio-carbonylamino)-1-(3,5-dichlorphenyl)-pyrrolidin-2,5-dion nach Anspruch 1.

18. Verfahren zur Herstellung von Verbindungen der Formel I, gekennzeichnet durch Kondensations-Reaktion eines substituierten Anilins II mit einem Asparaginsäure-Derivat III

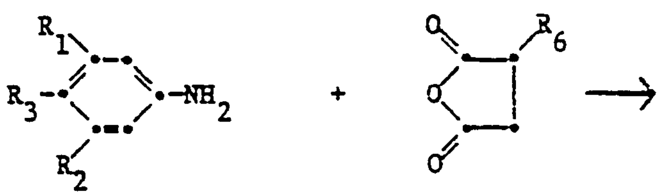

(I),

(II) (III)

worin $R_6$ für $-NH-CO-R_4$ oder $-NHR_7$ steht und $R_1$, $R_2$, $R_3$ und $R_4$ die unter Formel I angegebenen Bedeutungen haben, während $R_7$ für eine in der Peptid-Chemie übliche Schutzgruppe steht und, falls $R_6$ für $-NHR_7$ steht, die Reaktion über ein Zwischenprodukt der Formel IV

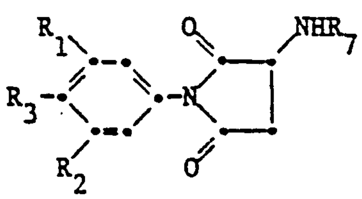

(IV)

verläuft, wobei die Schutzgruppe in an sich bekannter Weise abgespalten wird und die Aminogruppe mit einem für eine N-Acylierung reaktionsfähigen Carbonsäure-[Derivat] zu dem Endprodukt der Formel I reagiert.

19. Verfahren zur Herstellung von Verbindungen der Formel I, in denen $R_4$ einen Rest $-XR_5$ bedeutet, dadurch gekennzeichnet, dass man ein Asparaginsäurederivat der Formel V,

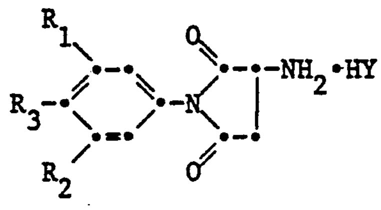

(V) ,

worin $R_1$ bis $R_3$ die unter Formel I, Anspruch 1, gegebene Bedeutung haben und Y für Halogen steht, in Gegenwart von zwei Aequivalenten eines säurebindenden Mittels mit Phosgen umsetzt und das entstehende Carbamoylchlorid der Formel VI

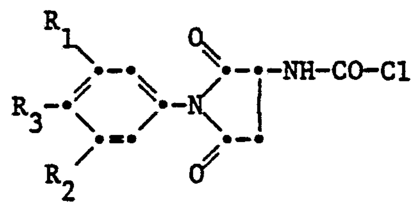

(VI)

in Gegenwart eines Aequivalentes säurebindenden Mittels mit einem Alkohol oder Thioalkohol der Formel VII,

$$HX-R_5 \qquad \text{(VII)}$$

worin X und $R_5$ die unter Formel I, Anspruch 1, gegebene Bedeutung haben, verestert.

20. Mittel zur Bekämpfung und/oder Verhütung eines Befalls durch phytopathogene Mikroorganismen, dadurch gekennzeichnet, dass es als mindestens eine aktive Komponente eine Verbindung der Formel I gemäss Anspruch 1 zusammen mit einem oder mit mehreren Trägerstoffen enthält.

21. Mittel nach Anspruch 20, dadurch gekennzeichnet, dass es als aktive Komponente eine Verbindung der Formel I gemäss einem der Ansprüche 2 bis 4 enthält.

22. Mittel nach Anspruch 20, dadurch gekennzeichnet, dass es als aktive Komponente eine Verbindung der Formel I gemäss einem der Ansprüche 5 bis 17 enthält.

23. Verwendung einer Verbindung der Formel I nach Anspruch 1 zur Bekämpfung und/oder Verhütung eines Befalles durch phytopathogene Mikroorganismen.

24. Verwendung nach Anspruch 23 von Verbindungen der Formel I gemäss einem der Ansprüche 2 bis 17.

25. Verwendung nach einem der Ansprüche 23 und 24, dadurch gekennzeichnet, dass es sich bei den Mikroorganismen um phytopathogene Pilze handelt.

26. Verwendung nach Anspruch 25, dadurch gekennzeichnet, dass es sich bei den Pilzen um Ascomycetes und/oder Fungi imperfecti handelt.